# EUROPÄISCHE PATENTSCHRIFT

(11) **EP 1 200 375 B1**
(45) Veröffentlichungstag und Bekanntmachung des Hinweises auf die Patenterteilung: **22.09.2004**
(21) Anmeldenummer: 00947931.2
(22) Anmeldetag: 05.07.2000
(51) Int. Cl.: C07B 43/02, C07C 201/08, C07D 217/02, C07D 307/77

(54) **NITRIERUNG IN EINEM STATISCHEN MIKROMISCHER**
NITRATION IN A STATIC MICROMIXER
NITRATION DANS UN MICROMELANGEUR STATIQUE

(30) Priorität: 29.07.1999 DE 19935692
(43) Veröffentlichungstag der Anmeldung: 02.05.2002
(73) Patentinhaber: MERCK PATENT GmbH, 64293 Darmstadt (DE)
(72) Erfinder: WURZIGER, Hanns, D-64291 Darmstadt (DE); STOLDT, Joeran, D-64331 Weiterstadt (DE); FABIAN, Kai, D-69259 Wilhelmsfeld (DE); SCHWESINGER, Norbert, D-98693 Ilmenau (DE)
(86) Internationale Anmeldenummer: PCT/EP2000/006317
(87) Internationale Veröffentlichungsnummer: WO 2001/009065

(56) Entgegenhaltungen:
- WO-A-99/22858

## Beschreibung

Die vorliegende Erfindung betrifft ein neues Verfahren zur Nitrierung von aromatischen und heteroaromatischen Verbindungen, in einem statischen Mikroreaktor mit sich kreuzenden Kanälen mit literaturbekannten sowie neuen Typen von Nitrierungsreagenzien.

Die Nitrierung von organischen Substraten ist in der chemischen Industrie ein sehr wichtiges und häufig durchgeführtes Verfahren. Zahlreiche Veröffentlichungen befassen sich mit diesem Thema.

Bei der Durchführung von Nitrierungen im technischen Maßstab bestehen besondere Sicherheitsprobleme, da einerseits mit zum Teil hochgiftigen chemischen Substanzen gearbeitet werden muß, welche für sich allein betrachtet bereits ein erhebliches Risiko für eine Umweltgefährdung darstellen. Andererseits verlaufen Nitrierungen im allgemeinen stark exotherm, so daß eine erhöhte Explosionsgefahr bei der Durchführung dieser Reaktionen besteht.

Durch die WO 99/22858 A1 wird u. a. die Nitrierung in einem Mikroreaktor beschrieben, wobei der Mikroreaktor in der einfachsten Ausführung aus einem einzigen dünnen Kanal im µ-Maßstab besteht, in den am Anfang zwei verschiedene Eingangskanäle münden und der sich am Ende in zwei Ausgangskanäle aufteilt. Für ein Scale-up müssen viele Kanäle mit den gleichen Merkmalen parallel geschaltet werden. In der Praxis ist dieses extrem aufwendig, wenn nicht gar unmöglich, da in einem solchen Fall in einer Vielzahl von parallelen Kanälen identische Strömungswiderstände realisiert werden müssen. Bereits wenn in einem solchen Fall nur ein Kanal einen anderen Widerstand aufweist, wirkt sich dieses auf das gesamte System aus und führt zu veränderten Bedingungen in allen Kanälen, wodurch wiederum eine veränderte Selektivität hervorgerufen wird. Die Nitrierung muss in einem nicht mischbaren 2-Phasen-System durchgeführt werden, insbesondere einem wässrig-organischen System, damit sich die Phasen am Ende wieder trennen können. Zu diesem Zweck muss sich eine stabile Grenzfläche zwischen den beiden Phasen bilden, damit ein kontinuierlicher Extraktionsprozess stattfinden kann.

Es hat bisher nicht an Versuchen gefehlt, diese Probleme, insbesondere diese Sicherheitsprobleme zu reduzieren. Sobald jedoch große Mengen nitrierter Produkte hergestellt werden sollen und ein Scale-up durchgeführt werden soll, treten die oben genannten Probleme, und insbesondere die Sicherheitsprobleme in den Vordergrund.

Aufgabe der vorliegenden Erfindung war es daher, ein einfach durchzuführendes, neues Verfahren zur Verfügung zu stellen, wodurch Nitrierungen von aromatischen und heteroaromatischen Verbindungen, in einfacher reproduzierbarer Weise mit erhöhter Sicherheit, mit vermindertem Restrisiko für eine Umweltgefährdung durchgeführt werden können. Aufgabe der Erfindung war es auch, entsprechend nitrierte Produkte in erhöhten Ausbeuten und verbesserten Reinheiten zur Verfügung zu stellen.

Die Lösung der Aufgabe erfolgt durch ein Verfahren zur Nitrierung von aromatischen oder heteroaromatischen Verbindungen, wobei man die aromatische oder heteroaromatische Verbindung in flüssiger Form oder in Lösung mit einem flüssigen oder gelösten Nitrierungsreagenz, ausgewählt aus der Gruppe verdünnte Salpetersäure, 100%-ige Salpetersäure, Kaliumnitrat in 100%-iger Schwefelsäure, Gemische aus Salpetersäure und Schwefelsäure ("Nitriersäure"), Salpetersäureester, Gemische von Salpetersäure mit anorganischen und organischen Anhydriden und Distickstoffpentoxid, kontinuierlich in einem statischen Mikroreaktor mit sich kreuzenden Kanälen während einer ausreichenden Verweilzeit intensiv vermischt und in homogener, flüssiger Phase reagieren läßt und aus dem entstandenen Reaktionsgemisch das erwünschte Nitrierungsprodukt isoliert.

Vorzugsweise handelt es sich bei dem zur Durchführung des Verfahrens verwendeten Mikroreaktor um einen temperierbaren Durchflußreaktor.

Eine besondere Variante des erfindungsgemäßen Verfahrens besteht darin, daß
a) die aromatische oder heteroaromatische Verbindung vorab derivatisiert wird
b) das erhaltene Derivat in einem Lösungsmittel gelöst wird,
c) mit einem Nitrierungsreagenz in einem Mikromischer nitriert wird und
d) das nitrierte Produkt aus der erhaltenen Lösung isoliert wird.

Vorzugsweise wird das nitrierte Produkt durch Extraktion mit einem Lösungsmittel aus dem Reaktionsgemisch abgetrennt, da dieses eine einfache Aufarbeitungsform darstellt.

Zur Durchführung des Verfahrens wird das Reaktionsgemisch so in den Mikroreaktor gepumpt, dass es diesen mit einer ausreichenden Durchflussgeschwindigkeit von 1 µl/min bis 10 ml/min durchströmt und die Reaktion bei einer Temperatur in einem Bereich von -40 bis 150 °C durchgeführt werden kann, wobei der Reaktionsverlauf gaschromatographisch verfolgt wird.

Vorzugsweise wird der Mikroreaktor vom Reaktionsgemisch mit einer Durchflussgeschwindigkeit von 5 µl/min bis 1 ml/min durchströmt und die Reaktion bei einer Temperatur in einem Bereich von -10 bis 80 °C durchgeführt.

Erfindungsgemäß wird das Reaktionsgemisch derart in den Mikroreaktor gepumpt, daß es diesen mit einer Durchflußgeschwindigkeit von mindestens 5µl/min durchströmt und die Reaktion bei einer Temperatur in einem Bereich von -10 bis 80 ° erfolgt. Gleichzeitig wird der Reaktionsverlauf gegebenenfalls gaschromatographisch verfolgt. Dieses kann gegebenenfalls kontinuierlich erfolgen.

Gegebenenfalls wird das erfindungsgemäße Verfahren durchgeführt, nachdem die aromatische oder heteroaromatische Verbindung in einem ersten Reaktionsschritt zu einem Carbamat umgesetzt worden ist.

Insbesondere werden nach dem erfindungsgemäßen Verfahren Verbindungen ausgewählt aus der Gruppe Toluol, 1,2,3,4-Tetrahydroisochinolin, N- Methoxycarbonyl-1,2,3,4-tetrahydroisochinolin und der Benzofuranderivate nitriert.

Aus verschiedenen Veröffentlichungen und Patentanmeldungen sind miniaturisierte Durchflußreaktoren für organische Reaktionen bekannt. Bevorzugt werden solche Reaktoren aus dünnen, miteinander verbundenen Siliziumstrukturen hergestellt. Solche miniaturisierten Reaktoren weisen sehr dünne Kanäle auf, welche an sich sehr leicht zum Verstopfen durch in der Reaktionslösung enthaltene oder gebildete Partikel neigen.

Die Voraussetzungen für die Durchführbarkeit einer Reaktion in solchen miniaturisierte Durchflußreaktoren bestehen daher darin, daß sie in homogener flüssiger Phase erfolgen kann und Ausfällungen oder die Bildung von Partikein während der Reaktion vermieden werden können.

Zur Lösung der vorliegenden Aufgabe wurden zahlreiche Versuche zur Nitrierung von organischen Verbindungen durchgeführt, insbesondere von aromatischen und heteroaromatischen Verbindungen.

Als Zwischenprodukt für die Synthese von chemischen Verbindungen, welche als Wirkstoffe zur Herstellung von Arzneimitteln verwendet werden, wird beispielsweise 8-Nitro-N-methoxycarbonyl-tetrahydroisochinolin benötigt. Diese Verbindung kann nach Methoden hergestellt werden, welche aus der Literatur bekannt sind; und zwar durch Nitrierung von 1,2,3,4-Tetrahydroisochinolin mit Kaliumnitrat in konzentrierter Schwefelsäure und nachfolgender Acylierung gemäß der im folgenden gegebenen allgemeinen Reaktionsgleichung:

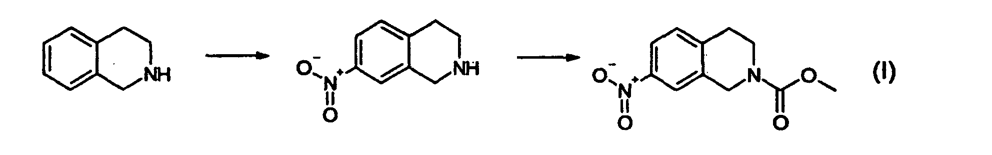

Es wurde im Rahmen des oben angesprochenen Versuchsprogramms versucht, diese Reaktion unter Zuhilfenahme eines Mikroreaktors durchzuführen. Aufgrund der Viskosität von 100%-iger Schwefelsäure baute sich jedoch im Mikromixer und in den dünnen Teflonschläuchen ein erheblicher Druck auf. Es wurde gefunden, daß unter Zuhilfenahme einer Einspritzpumpe, welche mit sehr geringer Pumpleistung arbeitet, dieses Problem überwunden werden kann.

Anschließende Versuche zeigten sehr effiziente Nitrierungsergebnisse. Nach erfolgter Reaktion wurde ein flüssiges Reaktionsgemisch erhalten, das vor der Abtrennung der gewünschten Produkte neutralisiert werden mußte.

Da das Ziel jedoch war, ein einfach und umweltfreundlich durchzuführendes Verfahren zur Verfügung zu stellen, wurde nach anderen Alternativen gesucht.

Es wurde überraschenderweise gefunden, daß durch eine veränderte Reihenfolge der Synthese dieses Problem umgangen werden kann.

In einem ersten Schritt wird 1,2,3,4-Tetrahydroisochinolin zu dem entsprechenden Carbamat umgesetzt. Das Carbamat wird in einem geeigneten Lösungsmittel, wie z. B. Dichtoromethan, gelöst und bei Umgebungstemperatur mit 65%-iger Salpetersäure nitriert.

Optimierungsversuche zeigten, daß diese Nitrierung bei einer Fließgeschwindigkeit von 5µl/min im Mikroreaktor mit einem guten Ergebnis, bezogen auf das Verhältnis der eingesetzten Ausgangsverbindungen zu erhaltenem Produkt, durchgeführt werden kann. Vorteilhafterweise kann das so erhaltene Nitrierungsprodukt aus der sauren Reaktionslösung direkt durch Extraktion isoliert werden.

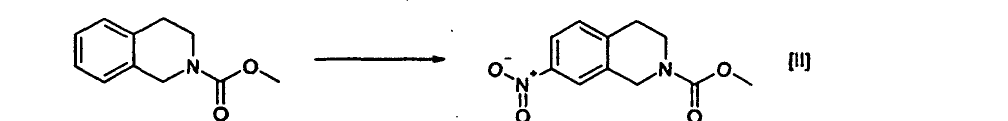

Das so gefundene Verfahren zur Durchführung der gewünschten Reaktion im durchströmten Mikroreaktor mit sich krenzenden Kanälen ist einer Reaktion im üblicherweise verwendeten gerührten Reaktor überlegen.

Ein ganz besonderer Vorteil ist, daß die Reaktion, sobald die Durchführungsparameter, wie Temperatur und Durchflußrate eingestellt sind und sich das System im Gleichgewicht befindet, die Reaktion so lange kontinuierlich durchgeführt werden kann, wie ein Bedarf an dem gewünschten Reaktionsprodukt besteht. Zudem kann das Produkt mit stabilen Ausbeuten in einer immer gleichen Qualität erhalten werden. Entsprechend eindrucksvolle Ergebnisse wurden beispielsweise bei der Nitrierung von N-Methoxycarbonyl-1,2,3,4-tetrahydroisochinolin in einer kontinuierlichen Reaktion erzielt, wobei letztere für die Dauer von sechs Tagen im Gleichgewicht gehalten wurde.

Es wurden auch die Einsatzmöglichkeiten des Mikroreaktors für weitere aromatische und heteroaromatische Verbindungen untersucht. Beispielhaft sei hier die Nitrierung von Toluol näher dargestellt.

In einer ersten Versuchsreihe wurde Toluol mit einer äquivalenten Menge Dichlormethan vermischt und bei verschiedenen Temperaturen mit 65%iger Salpetersäure bei verschiedenen Temperaturen nitriert. Das Verhältnis der nitrierten Isomere zu nicht umgesetztem Toluol ist in der folgenden Tabelle dargestellt. Die Daten beruhen auf Umsetzungen bei 10, 20 und 30 °C. Das Isomerenverhältnis wurde anhand von HPLC-Analysen ermittelt. Jedoch waren die HPLC-Signale nicht so sauber voneinander getrennt, daß die gebildete Menge des meta-Isomeren exakt hätte bestimmt werden können. Aus den erhaltenen Ergebnissen kann aber geschlossen werden, daß die erzielten Ergebnisse denen der Literatur entsprechen.

| **Temperatur [° C]** | **ortho-Nitrotoluol** | **para-Nitrotoluol** | **Toluol** |
|---|---|---|---|
| 0 | 41% | 22% | 37% |
| 20 | 59% | 40% | - |
| 30 | 59% | 41% | - |
| Literature² | 59% | 37% | |

Vergleichsweise wurde Toluol unverdünnt mit 100%iger Salpetersäure bei verschiedenen Temperaturen nitriert. Die Ergebnisse sind in der nachfolgenden Graphik dargestellt. Es kann daraus entnommen werden, daß mit steigender Temperatur der Anteil des ortho-Produkts sinkt, während die Bildung des para-substituierten Toluols und der in geringen Mengen gebildeten meta-Form zunimmt.

Unter der Voraussetzung, daß während der Passage durch die Mikrosysteme alle Substanzen in der gelösten flüssigen Phase bleiben und nicht ausfallen, können alle Nitrierungen mit den folgenden in der Literatur beschriebenen Nitrierungsreagenzien durchgeführt werden:
- Verdünnter Salpetersäure
- 100%-iger Salpetersäure
- Kaliumnitrat in 100%-iger Schwefelsäure
- Gemische aus Salpetersäure und Schwefelsäure ("Nitriersäure")
- Salpetersäureestern, allgemein
- Gemische von Salpetersäure mit anorganischen und organischen Anhydriden
- Distickstoffpentoxid

Die beschriebenen Nitrierungen verlaufen im statischen Mikromischer mit guten bis sehr guten Ausbeuten. Die Selektivität kann durch Variation verschiedener Parameter, wie beispielsweise Konzentration, Temperatur oder Verweilzeit, beeinflußt werden.

Der Vorteil der Nitrierung in Mikrofluidsystemen liegt im besseren Massen- und Wärmetransport, verbesserter Kontrolle der Reaktionszeit und der erhöhten Sicherheit. Hierfür verantwortlich sind die im System befindlichen sehr geringen Reagenzmengen.

Dieser Punkt ist besonders bei den i. a. stark exothermen Nitrierungen wichtig, wenn z. B. das sehr selektiv reagierende Gemisch bestehend aus 100%iger Salpetersäure und Essigsäureanhydrid - die reaktive Spezies ist das bei ca. 60°C explosionsartig zerfallende Acetylnitrat - eingesetzt wird.

Eine Änderung der Verweilzeit durch Variation der Pumpleistung zeigte in diesem Fall keinen bzw. nur einen geringfügigen Einfluß auf die Selektivität. Es ist auch anzunehmen, daß die Aktivität der Nitrierungsreagenzien und die Temperatur kaum das quantitative Ergebnis der Reaktion bei einer festgelegten Verweilzeit beeinflussen.

Dieser Effekt ist wesentlich stärker ausgeprägt, wenn eine Mischung aus Toluol und Essigsäure mit 100 %-iger Salpetersäure behandelt wird. In diesem Fall ist das reagierende Reagenz Acetylnitrat, das in Abhängigkeit von der zu nitrierenden Spezies teilweise als wesentlich reaktionsfähigeres aber auch selektiveres Nitrierungsreagenz als Salpetersäure allein angesehen wird.

Eine Mischung bestehend aus Salpetersäure und Essigsäureanhydrid oder vorab destilliertes Acetylnitrat sind ein kräftiges aber auch gefährliches Reagenz, da es sich oberhalb von 60 °C zersetzt.

Durch dieses Reagenz werden selbst Heterocyclen nitriert, welche sich sonst nur schwer nitrieren lassen. Aus verständlichen Gründen findet diese Methode normalerweise jedoch nur begrenzten Einsatz.

Anders als in üblicherweise verwendeten technischen Anlagen zur Durchführung von chemischen Reaktion läßt sich nach dem erfindungsgemäßen Verfahren in den erfindungsgemäßen Mikromischersystemen einerseits die Temperatur des Reaktionsgemischs in jedem Volumenelement konstant halten. Außerdem befinden sich zu jedem Zeitpunkt in der Vorrichtung nur sehr geringe Eduktmengen. Dieses bedeutet, daß im vorliegenden Mikromischersystem ohne weiteres die beschriebenen Nitrierungsreaktionen durchgeführt werden können, welche bisher nur unter besonderen und teuren Sicherheitsvorkehrungen erfolgen konnten.

In einfacher Weise lassen sich beispielsweise nach dem erfindungsgemäßen Verfahren Nitrierungen von Toluol, 1,2,3,4-Tetrahydroisochinolin, N-Methoxycarbonyl-1,2,3,4-tetrahydroisochinolin und von Benzofurandreivaten durchführen.

Weiterhin kommen als Aromaten alle mono- und polycyclischen, homo- oder heteroaromatischen Verbindungen, sowie Verbindungen, die ein mono- oder polycyclisches, homo- oder heteroaromatisches Grundsystem oder eine Teilstruktur, beispielsweise in Form von Substituenten aufweisen.

Insbesondere kommen als geeignete Aromaten in Betracht:
Benzol und dessen Derivate,
Naphthalin und dessen Derivate,
Azulen und dessen Derivate,
Anthracen und dessen Derivate,
Phenanthren und dessen Derivate,
Pyren und dessen Derivate,
Fluoren und dessen Derivate,
Chinone, wie beispielsweise ortho und para-Benzochinon und deren Derivate, alle dem Fachmann bekannten Naphthochinone und deren Derivate, Fluorenone, Anthrone, Phenanthrone, alle bekannten Anthrachinone und deren Derivate.

Als Heteroaromaten können eingesetzt werden:
sauerstoffhaltige, heteroaromatische Systeme (Furane), wie beispielsweise
benzanellierte Furane und deren Derivate,
Dibenzofurane und deren Derivate
Dibenzodioxane und deren Derivate,
Pyryliumkationen und deren Derivate,
Benzopyranone und deren Derivate
stickstoffhaltige heteroaromatische Systeme und deren Derivate, wie beispielsweise
Pyrrole, Pyrazole, Imidazole, Triazole, Tetrazole, Pyridine, Pyrazine, Pyrimidine, Pyridiniumsalze, Triazine, Tetrazine, Pyridin-N-oxid und deren Derivate benzanellierte Pyrrole (Indole, Carbazole, Benzimidazole, Benzotriazole) und deren Derivate
   Phenazin und deren Derivate
   Chinoline und Isochinoline,
   Cinnoline, Chinazoline, Chinoxaline,
   Phenanthroline und deren Derivate
   Bipyridyle und höhere Homologe
   Acridine, Acridone und deren Derivate
   Pyren und dessen Derivate
   als schwefelhaltige heteroaromatische Systeme und deren Derivate kommen beispielsweise in Frage
Thiophene und deren Derivate
benzanellierte Thiophene (Benzothiophene, Dibenzothiophene) und deren Derivate

Weiterhin sind Acenaphthylen, Thiazole, Isothiazole, Biphenylene, Purine, Benzothiadiazole, Oxazole und Isooxazole in dem erfindungsgemäßen Verfahren einsetzbar.

Als Lösungsmittel für diese Nitrierungen kommen in Frage:
- verdünnte und konzentrierte Säuren, wie beispielsweise Schwefelsäure, Salpetersäure, Essigsäure, Trifluoressigsäure
- Säureanhydride, wie beispielsweise Essigsäureanhydrid, Trifluoressigsäureanhydrid
- Gemische aus Säuren und Salzen wie z. B. Konzentrierte Schwefelsäure und KNO₃ und auch jede andere Kombination
- Halogenkohlenwasserstoffe, wie beispielsweise Chloroform, Tetrachlorkohlenstoff, Dichlormethan, Tetrachlorethan
- Ester, wie beispielsweise Essigsäueethylester
- Ether, wie beispielsweise Tetrahydrofuran, Diethylether, tert.-Butylmethylether
- Gemische der genannten Lösungsmittel jeglicher Art
- ionische Lösungsmittel, wie beispielsweise 1-Ethyl-3-methylimidazoliumtetrachloroaluminat, n-Butylpyridinium-tetrachloroaluminat oder 1-Ethyl-3-methylimidazolium-tetrafluoroborat.

Als Lösungsmittel für die Aufarbeitung/Extraktion kommen alle organischen Lösungsmittel in Frage, insbesondere:
- Ether, wie beispielsweise Diethylether, tert.-Butyl-methylether, Tetrahydrofuran etc.
- Halogenkohlenwasserstoffe, wie beispielsweise Chloroform, Tetrachlorkohlenstoff, Dichlormethan, Tetrachlormethan, Tetrachlorethen
- Alkohole, wie beispielsweise Methanol, Ethanol, 1- und 2-Propanol
- Nitrile, wie beispielsweise Acetonitril
- Ketone, wie beispielsweise Aceton
- Ester, wie beispielsweise Essigsäureethylester

Zur Durchführung der beschriebenen Nitrierungen sind Mikromischer geeignet, wie sie beispielsweise in WO 96/30113 A1 beschrieben sind. Geeignet sind aber auch einfacher ausgestaltete statische Mikromischer, worin eine ausreichend intensive Durchmischung der eingesetzten Flüssigkeiten in einfachen sich kreuzenden Kanälen erfolgt und eine ausreichende Verweilzeit des Reaktionsgemischs für die Reaktion im Reaktor gewährleistet ist.

Grundvoraussetzungen für die Einsetzbarkeit von Mikroreaktoren in dem erfindungsgemäßen Nitrierungsverfahren sind weiterhin:
- die Möglichkeit einer gleichmäßigen Temperierung in jedem Volumenelement des Reaktors,
- dichte und sichere Anschlußmöglichkeiten für Zu- und Ableitungen von Flüssigkeiten gegebenenfalls aber auch für weiteres Equipment zur Reaktionskontrolle oder für Analysezwecke,
- dichte Verbindung der den Mikroreaktor bildenden Einzelteile bzw. Strukturen sowohl innen als auch nach außen, so daß die Flüssigkeit-führenden Kanäle voneinander getrennt sind und keine Flüssigkeit nach außen austreten kann,
- leichte Handhabbarkeit bei Störungen.

Zum besseren Verständnis und zur Verdeutlichung der vorliegenden Erfindung werden im folgenden Beispiele gegeben, die im Rahmen des Schutzbereichs der vorliegenden Erfindung liegen, nicht jedoch geeignet sind, die Erfindung auf diese Beispiele zu beschränken.

### Beispiele

### Beispiel 1

1g 1,2,3,4-Tetrahydro-isochinolin wurden vorsichtig in 5 ml konzentrierter Schwefelsäure gelöst. Die Salpetersäure wurde hergestellt durch Lösen von 1g Kaliumnitrat in 5 ml konzentrierter Schwefelsäure. Zwei 2-ml-Einweg-Kunststoffspritzen wurden mit den beiden Lösungen gefüllt und an einer "Havard Apparatus pump 22" befestigt. Die Einwegspritzen selbst wurden mit einem statischen Siliziummischer verbunden, der wiederum mit einem dünnen, 80 cm langen Teflonschlauch mit einem Durchmesser von 0,25 mm verbunden war. Die Reaktion wurde bei Umgebungstemperatur mit einer Durchflußgeschwindigkeit von 5 µl/min durchgeführt. Das entstandene Reaktionsgemisch wurde in einem Gefäß gefüllt mit Eistückchen gesammelt und mit 2N NaOH neutralisiert bevor das gebildete Produkt mit Dichlormethan extrahiert wurde.

1g N-Methoxycarbonyl-1,2,3,4-Tetrahydro-isochinolin, gelöst in 5 ml Dichloromethan, wurde wie vorher beschrieben bei Umgebungstemperatur mit 65%iger Salpetersäure und einer Durchflußgeschwindigkeit von 5 µl/min nitriert. Die Aufarbeitung der organischen Phase erfolgte ohne vorherige Neutralisation.

Die gleichen Bedingungen wurden eingestellt für einen kontinuierlichen Versuch, der kontinuierlich über sechs Tage durchgeführt wurde. In diesem Fall wurden zwei Spritzen mit einem jeweiligen Fassungsvermögen von 50 ml verwendet.

### Beispiel 2

Eine Einwegplastikspritze mit einem Volumen von 2 ml gefüllt mit einer 1:1-Mischung bestehend aus Toluol und Dichlormethan. Eine zweite Spritze wurde gefüllt mit 65%-iger Salpetersäure. Diese beiden Spritzen wurden angeschlossen und eine Durchflußgeschwindigkeit von 5 µl/min eingestellt. Unter diesen Bedingungen wurde die Reaktion bei 0, 20 und 30 °C durchgeführt.

Der Reaktionsverlauf wurde mit einem Merck Hitachi HPLC-instrument (L 6200 pump, variable wavelength UV-detector and D 2500 chromato integrator) verfolgt und aufgezeichnet. Als Trennsäule wurde eine Merck Lichrocart® RP Select B 250/4 verwendet.
Lösungsmittel:
Gemisch aus 70% Acetonitril, 30 % Wasser, vermischt mit 1 % Trifluoressigsäure
Durchflußgeschwindigkeit: 0,6 ml/min
Detektorwellenlänge: 215 nm.

Die Nitrierungen von Toluol wurden mit Hilfe eines Hewlett-Packard 6890 series GC-System mit einem Massenselektionsdetektor HP 5973 verfolgt.

Zur Durchführung der Versuche mit 100 %iger Salpetersäure wurde eine Spritze mit Salpetersäure gefüllt und die andere mit reinem Toluol. Die Nitrierungen wurden bei 0, 21, 40, 60 and 80 °C durchgeführt.

Zur Durchführung der Versuche mit Acetylnitrat wurde eine Spritze mit einer 1:1 Mischung bestehend aus Toluol und Essigsäureanhydrid und eine andere mit 100%iger Salpetersäure gefüllt. Der Inhalt beider Spritzen wurde gleichzeitig zur intensiven Durchmischung und Nitrierung in zwei verschiedene Eingangskanäle eines Mikromischers gepumpt. Die Nitrierungen wurden bei -10, 0, 21, 40 and 60 °C durchgeführt, wobei der Reaktionsverlauf wie oben beschrieben verfolgt wurde.

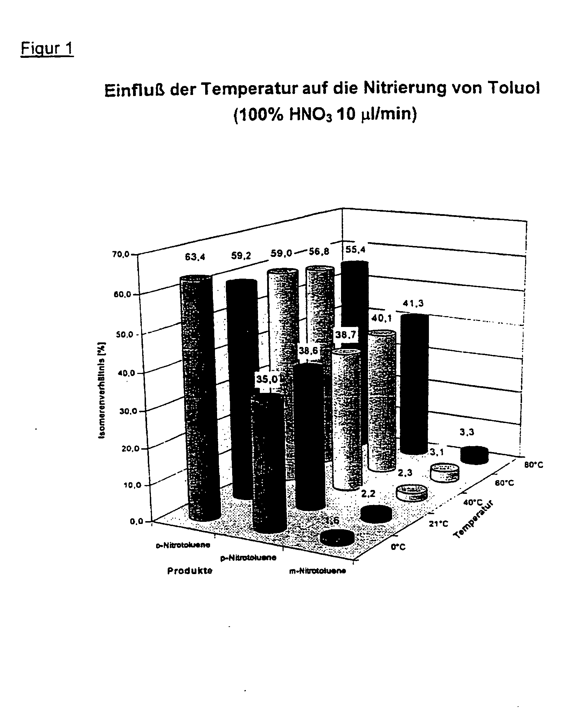

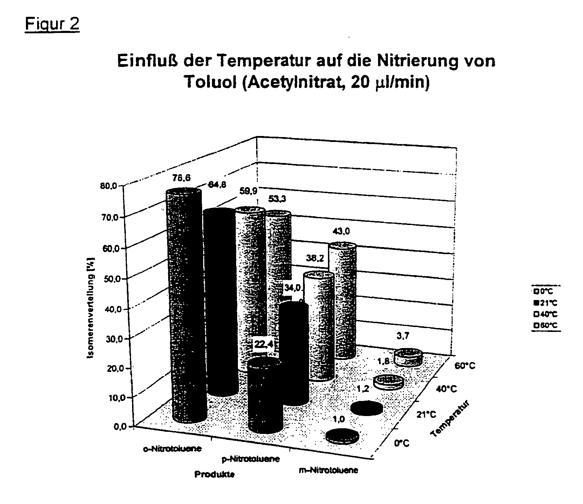

## Patentansprüche

1. Verfahren zur Nitrierung von aromatischen oder heteroaromatischen Verbindungen, **dadurch gekennzeichnet, dass**
die aromatische oder heteroaromatische Verbindung in flüssiger Form oder in Lösung mit einem flüssigen oder gelösten Nitrierungsreagenz, ausgewählt aus der Gruppe
verdünnte Salpetersäure, 100%-ige Salpetersäure, Kaliumnitrat in 100%iger Schwefelsäure, Gemische aus Salpetersäure und Schwefelsäure ("Nitriersäure"), Salpetersäureester, Gemische von Salpetersäure mit anorganischen und organischen Anhydriden und Distickstoffpentoxid, kontinuierlich in einem statischen Mikroreaktor mit sich kreuzenden Kanälen während einer ausreichenden Verweilzeit intensiv vermischt, in homogener, flüssiger Phase reagieren lässt und aus dem entstandenen Reaktionsgemisch das erwünschte Nitrierungsprodukt isoliert.

2. Verfahren gemäß Anspruch 1, **dadurch gekennzeichnet, dass** es sich bei dem verwendeten Mikroreaktor um einen temperierbaren Durchflussreaktor handelt.

3. Verfahren gemäß einem oder beiden der Ansprüche 1 bis 2, **dadurch gekennzeichnet, dass**
a) die aromatische oder heteroaromatische Verbindung vorab derivatisiert wird
b) das erhaltene Derivat in einem Lösungsmittel gelöst wird,
c) mit einem Nitrierungsreagenz in einem Mikromischer nitriert wird und
d) das nitrierte Produkt aus der erhaltenen Lösung isoliert wird.

4. Verfahren nach einem oder mehreren der Ansprüche 1 bis 3, **dadurch gekennzeichnet, dass** das nitrierte Produkt durch Extraktion mit einem Lösungsmittel aus dem Reaktionsgemisch abgetrennt wird.

5. Verfahren nach einem oder mehreren der Ansprüche 1 bis 4, **dadurch gekennzeichnet, dass** das Reaktionsgemisch den Mikroreaktor mit einer ausreichenden Durchflussgeschwindigkeit von 1 µl/min bis 10 ml/min durchströmt und die Reaktion bei einer Temperatur in einem Bereich von -40 bis 150 °C durchgeführt wird, wobei der Reaktionsverlauf gaschromatographisch verfolgt wird.

6. Verfahren gemäß Anspruch 5, **dadurch gekennzeichnet, dass** das Reaktionsgemisch den Mikroreaktor mit einer Durchflussgeschwindigkeit von 5 µl/min bis 1 ml/min durchströmt und die Reaktion bei einer Temperatur in einem Bereich von -10 bis 80 °C durchgeführt wird, wobei der Reaktionsverlauf gegebenenfalls kontinuierlich gaschromatographisch verfolgt wird

7. Verfahren gemäß einem oder mehreren der Ansprüche 3 bis 6, **dadurch gekennzeichnet, dass** eine aromatische oder heteroaromatische Verbindung in einem ersten Reaktionsschritt zu einem Carbamat umgesetzt wird.

8. Verfahren zur Nitrierung von aromatischen oder heteroaromatischen Verbindungen ausgewählt aus der Gruppe Toluol-, 1,2,3,4-Tetrahydroisochinolin-, N-Methoxycarbonyl-1,2,3,4-tetrahydroisochinolin- und der Benzofuranderivate gemäß einem oder mehreren der Ansprüche 1 bis 7.

9. Verfahren zur Herstellung von 8-Nitro-N-methoxycarbonyl-tetrahydroisochinolin nach einem Verfahren gemäß der Ansprüche 7 bis 8, **dadurch gekennzeichnet, dass** 1,2,3,4-Tetrahydroisochinolin in einem ersten Schritt zu N-Methoxycarbonyl-1,2,3,4-tetrahydroisochinolin umgesetzt wird, welches anschließend in Dichlormethan bei Umgebungstemperatur mit 65%iger Salpetersäure kontinuierlich in einem Mikroreaktor mit sich kreuzenden Kanälen nitriert wird.

10. Verfahren gemäß einem oder mehreren der Ansprüche 1 bis 8, **dadurch gekennzeichnet, dass** als Nitrierreagenz ein Gemisch aus Essigsäureanhydrid und 100%ige Salpetersäure oder vorab destilliertes Acetylnitrat verwendet wird.

11. Verfahren gemäß Anspruch 10, **dadurch gekennzeichnet, dass** Toluol als Edukt verwendet wird.

## Claims

1. Process for the nitration of aromatic or heteroaromatic compounds, **characterized in that** the aromatic or heteroaromatic compound, in liquid form or in solution, is vigorously mixed continuously in a static microreactor containing intersecting channels, for a sufficient residence time, with a liquid or dissolved nitrating reagent selected from the group comprising dilute nitric acid, 100% nitric acid, potassium nitrate in 100% sulphuric acid, nitric acid/sulphuric acid mixtures ("nitrating acid"), nitric acid esters, mixtures of nitric acid with inorganic and organic anhydrides, and dinitrogen pentoxide, the ingredients are allowed to react in a homogeneous liquid phase and the desired nitration product is isolated from the reaction mixture formed.

2. Process according to Claim 1, **characterized in that** the microreactor used is a continuous flow reactor capable of being thermostatted.

3. Process according to one or both of Claims 1 and 2, **characterized in that**
a) the aromatic or heteroaromatic compound is first derivatized,
b) the resulting derivative is dissolved in a solvent and
c) nitrated in a micromixer with a nitrating reagent, and
d) the nitrated product is isolated from the resulting solution.

4. Process according to one or more of Claims 1 to 3, **characterized in that** the nitrated product is separated from the reaction mixture by solvent extraction.

5. Process according to one or more of Claims 1 to 4, **characterized in that** the reaction mixture flows through the microreactor at a sufficient rate of 1 µl/min to 10 ml/min and the reaction is carried out at a temperature ranging from -40 to 150°C, the course of the reaction being monitored by gas chromatography.

6. Process according to Claim 5, **characterized in that** the reaction mixture flows through the microreactor at a rate of 5 µl/min to 1 ml/min and the reaction is carried out at a temperature ranging from -10 to 80°C, the course of the reaction optionally being monitored continuously by gas chromatography.

7. Process according to one or more of Claims 3 to 6, **characterized in that** an aromatic or heteroaromatic compound is converted to a carbamate in a first reaction step.

8. Process according to one or more of Claims 1 to 7 for the nitration of aromatic or heteroaromatic compounds selected from the group comprising derivatives of toluene, 1,2,3,4-tetrahydroisoquinoline, N-methoxycarbonyl-1,2,3,4-tetrahydroisoquinoline and benzofuran.

9. Preparation of 8-nitro-N-methoxycarbonyltetrahydroisoquinoline by a process according to Claims 7 and 8, **characterized in that** in a first step 1,2,3,4-tetrahydroisoquinoline is converted to N-methoxycarbonyl-1,2,3,4-tetrahydroisoquinoline, which is then reacted continuously in dichloromethane at ambient temperature with 65% nitric acid in a microreactor containing intersecting channels.

10. Process according to one or more of Claims 1 to 8, **characterized in that** the nitrating reagent used is a mixture of acetic anhydride and 100% nitric acid or previously distilled acetyl nitrate.

11. Process according to Claim 10, **characterized in that** toluene is used as the educt.

## Revendications

1. Procédé de nitration de composés aromatiques ou hétéroaromatiques, **caractérisé en ce que**
le composé aromatique ou hétéroaromatique sous forme liquide ou en solution avec un réactif de nitration dissout ou liquide, choisi parmi le groupe
acide nitrique dilué, acide nitrique à 100 %, nitrate de potassium dans de l'acide sulfurique à 100 %, mélanges à partir d'acide nitrique et d'acide sulfurique (« acide sulfonitrique »), ester d'acide nitrique, mélanges d'acide niyrique avec des anhydrides inorganiques et organiques et du pentoxyde de diazote,
est mélangé intensivement de façon continue dans un microréacteur statique à canaux croisés pendant un temps de station suffisant et réagit en phase liquide homogène et le produit de nitration souhaité est isolé du mélange réactionnel créé.

2. Procédé selon la revendication 1, **caractérisé en ce que** le microréacteur utilisé est un réacteur en flux traversant tempéré.

3. Procédé selon la revendication 1 ou 2 ou les deux, **caractérisé en ce que** :
a) le composé aromatique ou hétéroaromatique est dérivé au préalable,
b) le dérivé obtenu est dissout dans un solvant,
c) le dérivé obtenu est nitré avec un réactif de nitration dans un micromélangeur et
d) le produit nitré est isolé de la solution obtenue.

4. Procédé selon une ou plusieurs des revendications 1 à 3, **caractérisé en ce que** le produit nitré est séparé du mélange réactionnel par extraction avec un solvant.

5. Procédé selon une ou plusieurs des revendications 1 à 4, **caractérisé en ce que** le mélange réactionnel traverse le microréacteur avec un débit suffisant compris entre 1 µl/mn et 10 ml/mn et **en ce que** la réaction est réalisée à une température comprise entre -40 et 150°C, le déroulement de la réaction étant suivi par chromatographie.

6. Procédé selon la revendication 5, **caractérisé en ce que** le mélange réactionnel traverse le microréacteur avec un débit compris entre 5 µl/mn et 1 ml/mn et **en ce que** la réaction est réalisée à une température comprise entre -10 et 80°C, le déroulement de la réaction étant le cas échéant suivi de façon continue par chromatographie en phase gazeuse.

7. Procédé selon une ou plusieurs des revendications 3 à 6, **caractérisé en ce qu'**un composé aromatique ou hétéroaromatique est converti en carbamate dans une première étape de la réaction.

8. Procédé de nitration de composés aromatiques ou hétéroaromatiques choisis parmi le groupe toluol, 1,2,3,4-tétrahydroisoquinoléine, n-méthoxycarbonyl-1,2, 3,4-tétrahydroisoquinoléine et les dérivés de benzofuranne selon une ou plusieurs des revendications 1 à 7.

9. Procédé de préparation de 8-nitro-N-méthoxycarbonyltétrahydroisoquinoléine d'après un procédé selon les revendications 7 à 8, **caractérisé en ce que** la 1,2,3,4-tétrahydroisoquinoléine est convertie dans une première étape en N-méthoxycarbonyl-1,2,3,4-tétrahydroisoquinoléine, laquelle est ensuite nitrée de façon continue à température ambiante dans le dichlorométhane avec de l'acide nitrique à 65 % dans un microréacteur à canaux croisés.

10. Procédé selon une ou plusieurs des revendications 1 à 8, **caractérisé en ce que** le réactif de nitration utilisé est un mélange d'anhydride acétique et d'acide nitrique à 100 % ou un nitrate d'acétyle préalablement distillé.

11. Procédé selon la revendication 10, **caractérisé en ce que** du toluol est utilisé comme réactif.
